# EUROPEAN PATENT APPLICATION

(11) **EP 3 777 695 A1**
(43) Date of publication of application: **17.02.2021**
(21) Application number: 19290070.2
(22) Date of filing: 13.08.2019
(51) Int. Cl.: A61B 8/00, A61B 8/08

(54) **SYSTEMS AND METHODS FOR GUIDING THE ACQUISITION OF ULTRASOUND DATA**

(71) Applicant: Koninklijke Philips N.V., 5656 AG Eindhoven (NL)
(72) Inventor: Allain, Pascal, 5656 AE Eindhoven (NL); De Craene, Mathieu, 5656 AE Eindhoven (NL); Denis, Eric, 5656 AE Eindhoven (NL); Langet, Helene, 5656 AE Eindhoven (NL); Pizaine, Guillaume Julien Josep, 5656 AE Eindhoven (NL); Popoff, Alexandre, 5656 AE Eindhoven (NL); Van De Star, Sietze, 5656 AE Eindhoven (NL); Talgorn, Elise Claude Valentine, 5656 AE Eindhoven (NL); Villain, Nicolas François, 5656 AE Eindhoven (NL); Gauriau, Romane Isabelle Marie-Bernard, 5656 AE Eindhoven (NL); Piro, Paolo, 5656 AE Eindhoven (NL); Visweswara, Ashoka Sathanur, 5656 AE Eindhoven (NL); Saloux, Eric Michel Gerard Jean Pierre, 5656 AE Eindhoven (NL)
(74) Representative: de Haan, Poul Erik

(57) **Abstract**

The invention provides a method for guiding the acquisition of ultrasound data. The method includes obtaining aggregated ultrasound data, the aggregated ultrasound data comprising: ultrasound data of a given subject acquired by way of an ultrasound probe; and probe position data, wherein the probe position data relates to the location of the ultrasound probe in relation to the given subject when the ultrasound data was acquired. New ultrasound data is then acquired from the given subject by way of the ultrasound probe. Further new probe position data is acquired, wherein the new probe position data relates to the location of the ultrasound probe in relation to the given subject when the new ultrasound data is acquired. The new ultrasound data and the new probe position data is then compared to the aggregated ultrasound data and a guidance instruction is generated to provide to the user based on the comparison. The guidance instruction is then provided to the user.

## Description

### FIELD OF THE INVENTION

The invention relates to the field of ultrasound imaging, and more specifically to the field of guided ultrasound imaging.

### BACKGROUND OF THE INVENTION

Ultrasound imaging has become increasingly prevalent in recent years, particularly in clinical settings. Despite major advantages, such as non-invasiveness, safety and portability, ultrasound imaging suffers from a high dependence on operator skills, which may significantly affect the quality and homogeneity of examinations. Thus, the international ultrasound associations keep updating the guidelines for examination standards in order to homogenize ultrasound practice and improve the quality of care as described in Popescu et al, Updated standards and processes for accreditation of echocardiographic laboratories from The European Association of Cardiovascular Imaging. European Heart Journal - Cardiovascular Imaging (2014).

Ultrasound imaging is not a push-button technique. It requires an expert operator to acquire a good ultrasound image, meaning the repeatability and reproducibility of a given image is limited by its complexity. The complexity arises due to the localization step requiring the user to move the probe on the patient's body to reach the target location with the correct probe orientation while mentally integrating multiple images from the real-time ultrasound stream to reconstruct the patient's anatomy.

The achieved image quality is highly sensitive to the probe location and orientation, as well as to external, subject-dependent factors, including for instance the subject's position and breathing phase. Thus, the overall quality of an ultrasound exam heavily relies on the expertise and experience of the user performing the examination. It may take some time for a less experienced user to move the probe on the subject's body to obtain the desired image plane. The dependency on the user is one of the main sources of variations for establishing accurate quantitative ultrasound diagnoses. The dependency is particularly detrimental to the reproducibility of follow-up ultrasound examinations (examinations that are performed after an initial examination), making comparisons difficult.

In practice, the drawbacks induce limitations such as: poor (intra-user) repeatability and (inter-user) reproducibility because of non-reproducible views (foreshortening) and differences in image quality; a lack of standard protocols for scanning leading to long scan times and inefficient hospital workflow; and a dependency on user skill to perform the scan leading to long backlogs, delays, pressure on the user and poorer quality of care.

There is therefore a need for a means of ensuring the consistency of ultrasound imaging techniques.

### SUMMARY OF THE INVENTION

The invention is defined by the claims.

According to examples in accordance with an aspect of the invention, there is provided a computer-implemented method for guiding the acquisition of ultrasound data, the method comprising:
obtaining aggregated ultrasound data, the aggregated ultrasound data comprising:
   ultrasound data of a given subject acquired by way of an ultrasound probe;
   probe position data, wherein the probe position data relates to the location of the ultrasound probe in relation to the given subject when the ultrasound data was acquired;
   a feature (116) derived from the ultrasound data;
acquiring new ultrasound data from the given subject by way of the ultrasound probe;
acquiring new probe position data, wherein the new probe position data relates to the location of the ultrasound probe in relation to the given subject when the new ultrasound data is acquired;
deriving (135) a new feature from the new ultrasound data, which corresponds to the feature of the aggregated ultrasound data;
comparing the new ultrasound data, the new probe position data and the new feature to the aggregated ultrasound data;
generating a guidance instruction to provide to the user based on the comparison; and
providing the guidance instruction to the user.

The method provides for a means of performing multiple ultrasound examinations of a given region of interest in a consistent and reproducible manner. The method may be employed across separate ultrasound examinations or within the same ultrasound examination by utilizing ultrasound data obtained earlier within the same examination.

Typically, when repeat ultrasound measurements of the same region of interest are required, a sonographer, and in particular an unskilled sonographer, may struggle to obtain repeat measurements of a consistent quality. Inconsistent measurements may impair a physician's ability to care for a subject and so can be a limiting factor on the cases that ultrasound measurements can be relied upon.

By tracking the position of the probe relative to the subject being investigated, it is possible to associate the location of the probe with a given set of ultrasound data. The aggregated ultrasound data may then be compared to incoming ultrasound data and incoming probe position data in order to identify differences in the position of the probe or ultrasound data when compared to the previously acquired aggregated data.

Any differences between the aggregated data and the ultrasound data, the probe position data or both may be used to generate a guidance instruction to help the user recreate a previously acquired set of ultrasound data from a given position.

In this way, a user of any skill level may be guided to achieve a desired view, thereby increasing the consistency and accuracy of the obtained measurements.

Further, the user may be guided to acquire standard views, which are adjusted according to the morphology of the subject based on the aggregated ultrasound data. Thus, the acquired views may be standardized, in order to achieve a given clinical measurement, but also personalized to a given subject.

In an embodiment, the feature derived from the ultrasound data comprises an anatomical landmark identified within the ultrasound data and wherein the method further comprises:
deriving a new anatomical landmark within the new ultrasound data; and
comparing the new anatomical landmark with the anatomical landmark of the aggregated ultrasound data.

In this way, it is possible to generate a guidance instruction based on differences in anatomical landmarks, for example, by comparing a shape of an anatomical landmark derived from the new ultrasound data and the aggregated ultrasound data taking into account the position of the probe.

In an embodiment, the feature derived from the ultrasound data comprises a standard view score relating to the ultrasound data and wherein the method further comprises:
deriving a new standard view score relating to the new ultrasound data; and
comparing the new standard view score with the standard view score of the aggregated ultrasound data.

In this way, it is possible to ensure the quality of the ultrasound data is consistent across the various examinations.

In an embodiment, the aggregated ultrasound data comprises subject data and wherein the method further comprises:
obtaining new subject data; and
comparing the new subject data with the subject data of the aggregated ultrasound data.

In this way, information relating to the subject, such as age, gender and symptoms may be taken into account when performing the comparison and generating the guidance instruction.

In an embodiment, generating a guidance instruction comprises:
determining a difference between the aggregated ultrasound data and the new ultrasound data and the new probe position data; and
generating the guidance instruction based on the difference.

In this way, differences in the data may be identified and used as a basis for generating the guidance instruction.

In an embodiment, generating a guidance instruction further comprises:
obtaining user data, wherein the user data comprises a user experience level; and
adjusting the guidance instruction based on the user data.

In this way, data relating to the user performing the method maybe used to adjust the guidance instruction. For example, a less experienced user may require a more detailed guidance instruction; whereas, a more experienced user may require less detailed guidance.

In an embodiment, generating a guidance instruction comprises:
determining a morphological change in the given subject based on the comparison; and
adjusting the guidance instruction based on the morphological change.

In this way, morphological changes within the subject may be accounted for when generating the guidance instruction. For example, the morphological change may be a temporary change, such as different phases within a breath cycle, or a permanent change, for example resulting from a treatment or an intervention.

In an embodiment, the method further comprises adjusting an imaging parameter of the ultrasound probe based on the comparison.

In this way, an imaging parameter of the ultrasound probe may be adjusted in order to bring the new ultrasound data into line with the aggregated ultrasound data.

In an embodiment, the comparison and generating the guidance is performed by way of a machine learning algorithm.

In an embodiment, the guidance instruction comprises one or more of:
a visual instruction;
an audible instruction;
a tactile instruction; and
a digital instruction.

In an embodiment, the guidance instruction is a descriptive instruction and/or a prescriptive instruction.

According to examples in accordance with an aspect of the invention, there is provided a computer program comprising computer program code means which is adapted, when said computer program is run on a computer, to implement the method described above.

According to examples in accordance with an aspect of the invention, there is provided an ultrasound imaging system, the system comprising:
a processor adapted to:
   obtain aggregated ultrasound data, the aggregated ultrasound data comprising:
   ultrasound data of a given subject acquired by way of an ultrasound probe;
   probe position data, wherein the probe position data relates to the location of the ultrasound probe in relation to the given subject when the ultrasound data was acquired; and
   a feature (116) derived from the ultrasound data;
an ultrasound probe adapted to acquire new ultrasound data from the given subject by way of the ultrasound probe; and
a guidance unit adapted to acquire new probe position data, wherein the new probe position data relates to the location of the ultrasound probe in relation to the given subject when the new ultrasound data is acquired,
wherein the processor is further adapted to:
   derive a new feature from the new ultrasound data, which corresponds to the feature of the aggregated ultrasound data;
   compare the new ultrasound data, the new probe position data and the new feature to the aggregated ultrasound data;
   generate a guidance instruction to provide to the user based on the comparison; and
   provide the guidance instruction to the user.

In an embodiment, the guidance unit comprises one or more of:
a camera tracking system;
an inertial measurement unit;
a smart paper system;
a local positioning system; and
a beacon based tracking system.

The guidance unit may comprise any suitable tracking system for acquiring the probe position data.

In an embodiment, the system further comprises one or more of:
a visual indicator, wherein the guidance instruction comprises a visual instruction;
a speaker, wherein the guidance instruction comprises an audible instruction;
a vibration unit, wherein the guidance instruction comprises a tactile instruction; and
a digital display, wherein the guidance instruction comprises a digital instruction.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows an ultrasound diagnostic system to explain the general operation;
Figure 2 shows a method of the invention;
Figure 3 shows an example of visual feedbacks and guidance instructions that may be provided to the user; and
Figure 4 shows a further example of feedback and guidance instructions that may be provided to the user using information from previous exams.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for guiding the acquisition of ultrasound data. The method includes obtaining aggregated ultrasound data, the aggregated ultrasound data comprising: ultrasound data of a given subject acquired by way of an ultrasound probe; probe position data, wherein the probe position data relates to the location of the ultrasound probe in relation to the given subject when the ultrasound data was acquired; and a feature derived from the ultrasound data. New ultrasound data is then acquired from the given subject by way of the ultrasound probe and a new feature is derived from the new ultrasound data. Further new probe position data is acquired, wherein the new probe position data relates to the location of the ultrasound probe in relation to the given subject when the new ultrasound data is acquired. The new ultrasound data, the new probe position data and the new feature is then compared to the aggregated ultrasound data and a guidance instruction is generated to provide to the user based on the comparison. The guidance instruction is then provided to the user.

The method provides for a means of personalizing an ultrasound examination to a given subject and of improving the repeatability and reproducibility of said examination, thereby ensuring that the ultrasound data acquired from the subject by the same operator at different times, or by different operators in comparable conditions, will be of the same quality. In a very general setting, this can be accomplished by providing explicit assistance to the user during the scan by way of the guidance instruction, in order to directly or indirectly guide them to the best possible data acquisition.

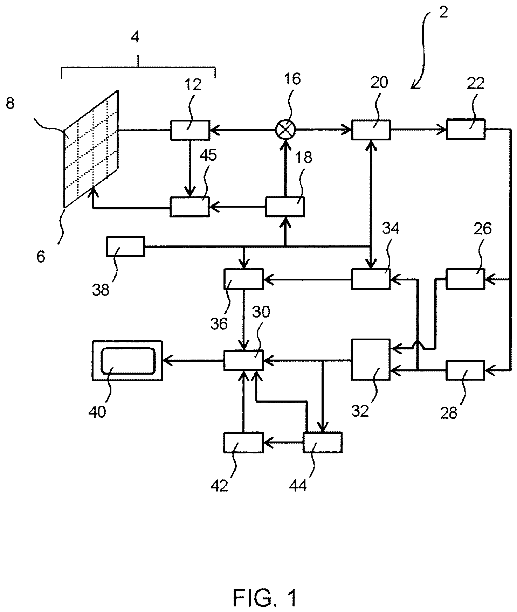

As the above method may be employed in an ultrasound imaging system, the general operation of an exemplary ultrasound system will first be described, with reference to Figure 1, and with emphasis on the signal processing function of the system since this invention relates to the processing of the signals measured by the transducer array.

The system comprises an array transducer probe 4 which has a transducer array 6 for transmitting ultrasound waves and receiving echo information. The transducer array 6 may comprise CMUT transducers; piezoelectric transducers, formed of materials such as PZT or PVDF; or any other suitable transducer technology. In this example, the transducer array 6 is a two-dimensional array of transducer elements 8 capable of scanning either a 2D plane or a three dimensional volume of a region of interest. In another example, the transducer array may be a 1D array.

The transducer array 6 is coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

It should be noted that the microbeamformer is entirely optional. Further, the system includes a transmit/receive (T/R) switch 16, which the microbeamformer 12 can be coupled to and which switches the array between transmission and reception modes, and protects the main beamformer 20 from high energy transmit signals in the case where a microbeamformer is not used and the transducer array is operated directly by the main system beamformer. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below. Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Figure 1 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor produces measures of different flow conditions such as the volume rate of blood flow in addition to structural measurements such as the sizes of organs and gestational age. The quantification processor may receive input from the user control panel 38, such as the point in the anatomy of an image where a measurement is to be made.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

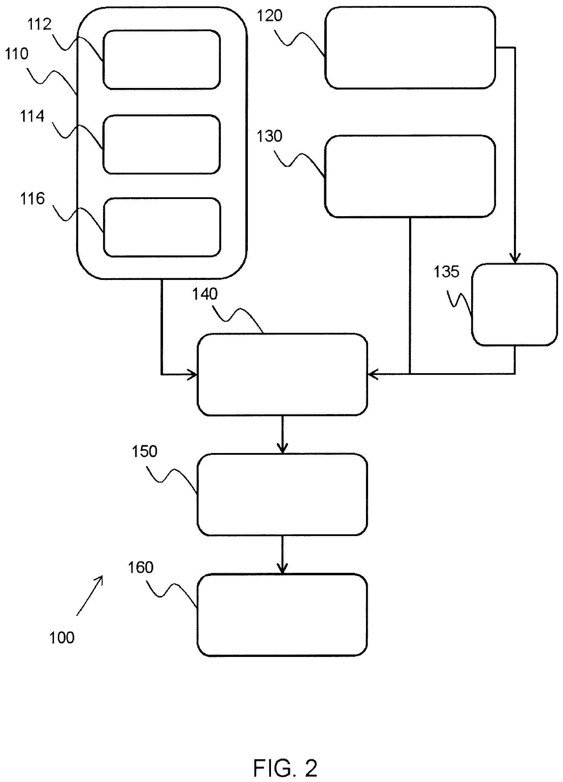

Figure 2 shows a method 100 for guiding the acquisition of ultrasound data.

In step 110, aggregated ultrasound data is obtained.

The aggregated ultrasound data comprises ultrasound data 112 of a given subject acquired by way of an ultrasound probe and probe position data 114, wherein the probe position data relates to the location of the ultrasound probe in relation to the given subject when the ultrasound data was acquired. The aggregated ultrasound data further comprises a feature derived from the ultrasound data. The feature may be an anatomical landmark, an image quality score, an image recognition score and the like.

The aggregated ultrasound data may comprise data from any number of previous ultrasound investigations. Alternatively, the aggregated ultrasound data may comprise data obtained within the same data acquisition session.

In step 120, new ultrasound data is acquired from the given subject by way of the ultrasound probe and in step 130, new probe position data is acquired. The new probe position data relates to the location of the ultrasound probe in relation to the given subject when the new ultrasound data is acquired.

The new ultrasound data is used to derive a new feature, which corresponds to the feature of aggregated ultrasound data. In other words, if the feature of the aggregated ultrasound data is an anatomical feature, the new feature derived from the new ultrasound data will also be an anatomical feature for the purposes of comparison. The feature may include multiple features or combinations of features.

For example, the probe position data may be acquired by way of a positioning system based on one or more sensors adapted to track the position and orientation of the ultrasound probe in real-time with respect to the subject's body. The new position data and the new ultrasound data may be aggregated on a computational device adapted to carry out the method.

Put another way, an ultrasound scan is performed on a subject using an ultrasound system that includes an on-/off-body localization sensor system with on-/off-body sensors adapted to capture the location information of the ultrasound probe during the scan of the subject.

During the scan, the location and orientation information from the sensor system in communication with the probe is recorded and stored. Position data captured by the localization sensor system may then be coupled with the real-time ultrasonic images. This may be performed by way of an artificial intelligence algorithm. In this way, the location of the probe on the patient's body required to achieve a given view may be determined, thereby allowing the user to consistently explore and quantify an area of interest.

The recorded ultrasound data and probe position stream data, as well as other data such as anatomical landmarks detected by a machine learning algorithm, may be aggregated and archived as part of the aggregated ultrasound data for improving future acquisitions.

The data relating to the anatomical landmark may contain derived information or measures that can be calculated from it. For example, in a cardiac application, the automatic identification and segmentation of a ventricle may be used to calculate clinically relevant values such as the length of the ventricle long axis, the ventricle volume, the ejection fraction, and the like. This data may then be used in the comparison between the aggregated data and the new data.

When the new ultrasound data and the new probe position data are acquired, the same localization sensor system with on-/off-body sensors may be set up to capture the location and orientation information of the ultrasound probe during a follow-up scan of a patient.

In step 140, the new ultrasound data, the new probe position data and new feature are compared to the aggregated ultrasound data.

The new ultrasound data and the new probe position data may be obtained using the same, or a similar, ultrasound probe and localization sensor system as described above.

The probe localization system consists of sensors positioned either on body or outside the body of the subject, which may be used to accurately locate the probe position relative to the body of the subject.

There are various techniques that are available for performing accurate probe localization on the human body. Off-body localization systems may include camera-based system that capture the image and then use image processing algorithms to determine the location of the probe on the body. On-body localization systems may include various beacon-based techniques where a beacon present on a fixed location on the body can be used as a reference point to find the location of the probe with respect to this fixed beacon. The beacon may comprise a radio frequency (RF) beacon, a magnetic field beacon, a body channel sensing beacon and the like. The sensor may include a 6 degrees of freedom (DOFs) sensor placed on the probe to compute the orientation of the probe.

In addition, the aggregated ultrasound data may further include an anatomical landmark identified within the ultrasound data. In this case the method may further include identifying a new anatomical landmark within the new ultrasound data and comparing the new anatomical landmark with the anatomical landmark of the aggregated ultrasound data.

The anatomical landmark may be identified within the ultrasound data using any suitable means, such as a segmentation or image recognition algorithm implemented by way of a machine learning algorithm.

In addition, the aggregated ultrasound data may further include a standard view score relating to the ultrasound data. In this case the method further comprises may further include calculating a new standard view score relating to the new ultrasound data and comparing the new standard view score with the standard view score of the aggregated ultrasound data.

In other words, the quality of the ultrasound data may be assessed using a numerical score, the standard view score, as part of the comparison. In this way, the quality of the ultrasound data may be monitored and be used to guide the user to acquire higher quality images in order to maintain a consistent quality across repeat measurements.

It should be noted that the numerical score may include multiple numerical scores, or combinations of numerical scores. Further, the standard view score may be a categorical score, such as poor, good, very good and the like. In addition, the standard view score may include any type of rating system, such as a color scale indicating the quality of the incoming data.

Further, the aggregated ultrasound data may further comprise subject data. In this case, the method may further include obtaining new subject data and comparing the new subject data with the subject data of the aggregated ultrasound data.

The subject data may be any piece of information relating to the subject, such as: age; gender; position during examination; breathing phase; heart rate; recent changes in morphology, such as operations; and the like.

Thus, changes in the subject between ultrasound examinations may be accounted for in the comparison between the aggregated ultrasound data and the new ultrasound data.

Further, the aggregated ultrasound data may also include data pertaining to the ultrasound equipment being used. For example, the make and model of the ultrasound system as well as imaging data such as: gain; imaging depth; additional software processing of the ultrasound data; and the like, may be included in the aggregated ultrasound data for use in the comparison with the incoming ultrasound data.

As stated above, the comparison may be implemented using an artificial intelligence framework. The artificial intelligence framework may rely on supervised machine learning algorithms taking as input the probe position and/or orientation, the incoming ultrasound data and/or information from previous exams on the same patient. Put another way, the machine learning algorithms may take any of the inputs described above in order to perform the comparison between the aggregated ultrasound data and the new ultrasound data and the new probe position data.

In step 150, a guidance instruction is generated to provide to the user based on the comparison.

The guidance instruction may include a variety of information depending on the data provided as input to the comparison step described above. For example, the guidance may include: a displacement in position and/or orientation of the probe with respect to a reference position, recorded either from the current or a previous ultrasound examination; a medically-relevant image quality score the quality of the new ultrasound data, for example compared to previously acquired ultrasound data; standard view type classification score indicating, for example by comparing the new ultrasound data to a standardized reference image for investigation of a given area; a segmentation of an anatomical structure of interest as compared to a previous segmentation of the same anatomical structure; an automatic quantification of a physiological indicator, such as volumes, lengths, areas of anatomical structures or any combination of these, indicators based on motion analysis and the like; and comparisons with aggregated ultrasound data from previous ultrasound examinations on the same patient

As described above, the comparison of the aggregated ultrasound data and the new ultrasound data and the new probe position data may highlight differences between the data sets. For example, the probe position comparison may reveal that the ultrasound probe is in a different location and/or orientation to a previous examination, or earlier data acquisition of the same examination. In this case, the guidance instruction may include an instruction to move the probe such that it occupies the previous position, thereby ensuring that repeat data measurements may be taken from the same place.

Further, the guidance instruction may be generated based on user data, such as the user's experience level. In other words, the guidance instruction may be adjusted in order to compensate for a lack of experience of the user, thereby ensuring consistent measurements regardless of the experience level of the user.

Where the comparison included the comparison of subject data, the guidance instruction may be generated based on the clinical history of the subject. In this way, the guidance instruction may guide the user to ensure that all ultrasound data for covering the anatomical/functional extent of the pathology are acquired. Further, the guidance instruction may recommend, for example by way of a machine learning algorithm, various additional views, or probe positons for data acquisition, based on the clinical history of the subject or where the comparison reveals an abnormal motion, flow or anatomy.

Put another way, data from the probe positioning system and results of the comparison of the aggregated ultrasound data and the new ultrasound data are combined to provide high-level guidance-oriented outputs, which may be provided as information to the user and/or to the system. User feedback can be descriptive (for example, a real-time visual indication of the image quality) and/or prescriptive (for example, a recommendation of actions to take to change the probe location to reach an optimal position). System feedback may take the form of an automatic instruction, such as the automatic adjustment of imaging parameters to match those preciously used in a given location or examination.

In other words, the comparison may be used to generate a guidance instruction for guiding the user to reproduce the views from previous exams, or earlier acquisitions of the same exam, by guiding probe orientations and/or positons to those contained in the aggregated ultrasound data; whilst, possible compensating for a difference in expertise between the users performing previous and current examinations and/ or adjusting for possible morphological changes by using machine learning algorithms to find the closest possible view in the vicinity of the reference view.

In step 160, the guidance instruction is provided to the user. The guidance instruction may include one or more of: a visual instruction; an audible instruction; a tactile instruction; and a digital instruction.

The methods described above may be executed on the same computational device as the ultrasonic recording device, the ultrasound system, or on a different one, such as in a cloud processing environment.

By integrating real-time image feedback through AI with probe orientation and localization, the user may be guided by, for example: visualizing all regions explored by the ultrasound probe, identifying those regions with the highest image quality; automatically standardizing the acquisition of ultrasound data with respect to. previous exams by recalling the imaging parameters, such as imaging views, anatomical landmarks and the like; and providing a checklist of acquisitions to be performed for the subject under investigation based on the findings of a previous exam, or an earlier acquisition of the same exam.

The present invention overcomes the limitations inherent to an ultrasonic guidance method based on image feedback only. By accessing directly the probe position through the probe positioning system as described above, ambiguities when reconstructing probe position/orientation from the image only are eliminated.

Such a reconstruction is typically impacted by morphological variability of the subject. By personalizing the acquisition for the subject under investigation as described above, the ultrasound examination is less impacted by the limitations of an average model or protocol. Although personalized to the needs of the subject, image quality and views may be standardized across subjects and repeat examinations of the same subject, thereby improving reproducibility and repeatability of the acquired measurements.

The method provides a means to guide a non-expert user in a simple, patient-specific way to acquire an ultrasound image while verifying and standardizing the image quality of the image acquisition.

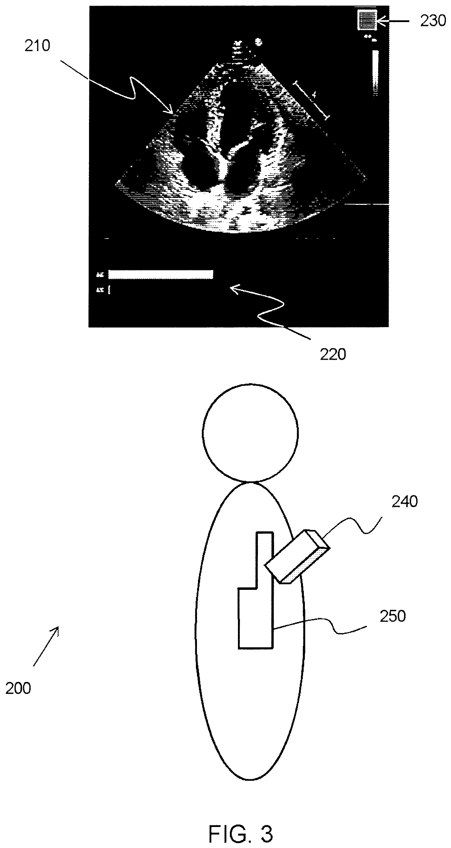

Figure 3 shows an example 200 of visual feedbacks and guidance instructions that may be provided to the user.

The real ultrasonic image stream 210 may be augmented with outputs from the comparison, which includes in this example, a real-time cardiac view recognition score 220 (which is an indicator of similarity between the current image and a given reference image) and an image quality score 230. This information may be combined with the recorded probe position information 240 to provide a map 250 of the subject's body areas explored by the user alongside a score corresponding to the best image obtained.

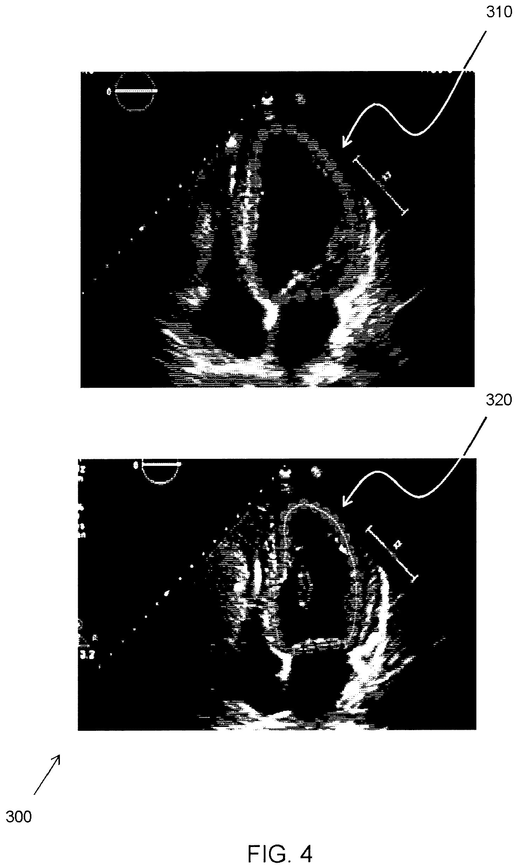

Figure 4 shows a further example 300 of feedback and guidance instructions that may be provided to the user using information from previous exams.

In this example, an anatomical landmark 310 has been identified and segmented during a previous examination, and is displayed along with the same anatomical landmark as identified in real-time 320 from the incoming data, for comparison.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A computer-implemented method (100) for guiding the acquisition of ultrasound data, the method comprising:
obtaining (110) aggregated ultrasound data, the aggregated ultrasound data comprising:
ultrasound data (112) of a given subject acquired by way of an ultrasound probe;
probe position data (114), wherein the probe position data relates to the location of the ultrasound probe in relation to the given subject when the ultrasound data was acquired; and
a feature (116) derived from the ultrasound data;
acquiring (120) new ultrasound data from the given subject by way of the ultrasound probe;
acquiring (130) new probe position data, wherein the new probe position data relates to the location of the ultrasound probe in relation to the given subject when the new ultrasound data is acquired;
deriving (135) a new feature from the new ultrasound data, which corresponds to the feature of the aggregated ultrasound data;
comparing (140) the new ultrasound data, the new probe position data and the new feature to the aggregated ultrasound data;
generating (150) a guidance instruction to provide to the user based on the comparison; and
providing (160) the guidance instruction to the user.

2. A method as claimed in claim 1, wherein the feature (116) of derived from the ultrasound data comprises an anatomical landmark identified within the ultrasound data and wherein the method further comprises:
deriving a new anatomical landmark within the new ultrasound data; and
comparing the new anatomical landmark with the anatomical landmark of the aggregated ultrasound data.

3. A method as claimed in any of claims 1 to 2, wherein the feature (116) of derived from the ultrasound data comprises a standard view score relating to the ultrasound data and wherein the method further comprises:
deriving a new standard view score relating to the new ultrasound data; and
comparing the new standard view score with the standard view score of the aggregated ultrasound data.

4. A method as claimed in any of claims 1 to 3, wherein the aggregated ultrasound data (110) comprises subject data and wherein the method further comprises:
obtaining new subject data; and
comparing the new subject data with the subject data of the aggregated ultrasound data.

5. A method as claimed in any of claims 1 to 4, wherein generating (150) a guidance instruction comprises:
determining a difference between the aggregated ultrasound data and the new ultrasound data and the new probe position data; and
generating the guidance instruction based on the difference.

6. A method as claimed in any of claims 1 to 5, wherein generating (150) a guidance instruction further comprises:
obtaining user data, wherein the user data comprises a user experience level; and
adjusting the guidance instruction based on the user data.

7. A method as claimed in any of claims 1 to 6, wherein generating (150) a guidance instruction comprises:
determining a morphological change in the given subject based on the comparison; and
adjusting the guidance instruction based on the morphological change.

8. A method as claimed in any of claims 1 to 7, wherein the method (100) further comprises adjusting an imaging parameter of the ultrasound probe based on the comparison.

9. A method as claimed in any of claims 1 to 8, wherein the comparison (140) and generating (150) the guidance is performed by way of a machine learning algorithm.

10. A method as claimed in any of claims 1 to 9, wherein the guidance instruction comprises one or more of:
a visual instruction;
an audible instruction;
a tactile instruction; and
a digital instruction.

11. A method as claimed in any of claims 1 to 10, wherein the guidance instruction is a descriptive instruction and/or a prescriptive instruction.

12. A computer program comprising computer program code means which is adapted, when said computer program is run on a computer, to implement the method of any of claims 1 to 11.

13. An ultrasound imaging system (2), the system comprising:
a processor adapted to:
obtain aggregated ultrasound data, the aggregated ultrasound data comprising:
ultrasound data of a given subject acquired by way of an ultrasound probe;
probe position data, wherein the probe position data relates to the location of the ultrasound probe in relation to the given subject when the ultrasound data was acquired; and
a feature (116) derived from the ultrasound data;
an ultrasound probe (4) adapted to acquire new ultrasound data from the given subject by way of the ultrasound probe; and
a guidance unit adapted to acquire new probe position data, wherein the new probe position data relates to the location of the ultrasound probe in relation to the given subject when the new ultrasound data is acquired,
wherein the processor is further adapted to:
derive a new feature from the new ultrasound data, which corresponds to the feature of the aggregated ultrasound data;
compare the new ultrasound data, the new probe position data and the new feature to the aggregated ultrasound data;
generate a guidance instruction to provide to the user based on the comparison; and
provide the guidance instruction to the user.

14. A system (2) as claimed in claim 13, wherein the guidance unit comprises one or more of:
a camera tracking system;
an inertial measurement unit;
a smart paper system;
a local positioning system; and
a beacon based tracking system.

15. A system (2) as claimed in any of claims 13 to 14, wherein the system further comprises one or more of:
a visual indicator, wherein the guidance instruction comprises a visual instruction;
a speaker, wherein the guidance instruction comprises an audible instruction;
a vibration unit, wherein the guidance instruction comprises a tactile instruction; and
a digital display, wherein the guidance instruction comprises a digital instruction.
